# EUROPEAN PATENT APPLICATION

(11) **EP 4 458 254 A1**
(43) Date of publication of application: **06.11.2024**
(21) Application number: 22916626.9
(22) Date of filing: 23.12.2022
(51) Int. Cl.: A61B 5/00, A61B 5/11, A41D 1/00, A41D 13/00

(54) **SMART WEARABLE CLOTHING INCLUDING STRETCHABLE CIRCUIT ELECTRODE AND SMART WEARABLE SYSTEM**

(30) Priority: 28.12.2021 KR 20210190335; 20.12.2022 KR 20220179095
(71) Applicant: Midas H&T Inc., Pohang-si Gyeongsangbuk-do 37673 (KR)
(72) Inventor: CHANG, Se Yun, Gwacheon-si Gyeonggi-do 13818 (KR); CHO, Hye Yeon, Seoul 08202 (KR); PARK, Hye Ji, Pohang-si Gyeongsangbuk-do 37761 (KR)
(74) Representative: Carpmaels & Ransford LLP
(86) International application number: PCT/KR2022/021178
(87) International publication number: WO 2023/128484

(57) **Abstract**

The present invention relates to smart wearable clothing including a stretchable circuit electrode and a smart wearable system, and the smart wearable clothing according to an embodiment of the present invention comprises: a fabric; a stretchable circuit electrode formed on the fabric; a stretchable ACF formed on the circuit electrode; and a chipset formed on the stretchable ACF.

## Description

### TECHNICAL FIELD

The present disclosure relates to smart wearable clothing including a stretchable circuit electrode, and a smart wearable system.

### BACKGROUND ART

Today, with the development of IT technology, wearable devices that are wearable electronic devices, appear and wearable technology is further developed. In particular, as IT convergence technology has emerged as a key topic in the global market, a demand for new concept smart clothing technology for providing diverse and creative services even in the smart clothing field is increasing.

Wearable devices need to be foldable and bendable to fit bodies due to elasticity, excellent durability, and excellent strain rate thereof. In this regard, stretchable electronic devices are attracting attention as promising next-generation electronic devices. Stretchable electronic devices are devices capable of maintaining functions even when stretched, and intensive research is being conducted in academia and industry on new mechanically durable materials and deformable conductors and circuits, new processing methods, the development of elastic materials for stretchable substrates, and the like.

Among them, a stretchable electrode is a basic component of a stretchable electronic device and has previously been manufactured by forming a conductive layer on a film-type stretchable elastomer substrate using a metal ink printing method. In addition, for commercialization of wearable devices, issues such as physical and electrical interfaces between complex devices and layers need to be solved. Mismatch in the modulus of elasticity between hard layers and soft layers may cause delamination and cracking during repeated deformation, resulting in a reduction in device performance. To solve these issues, an anisotropic conductive film (ACF) may be used in an electrical interconnection method of a rigid electronic device, which has a simple manufacturing process and guarantees both mechanical and electrical connections, thereby making it suitable for soft electronic applications, and includes a composite of a polymer and metal particles. However, in this field of ACF research, it has not been used in smart wearable clothing.

The above description has been possessed or acquired by the inventor(s) in the course of conceiving the present disclosure and is not necessarily an art publicly known before the present application is filed.

### DISCLOSURE OF THE INVENTION

### TECHNICAL GOALS

To solve the above-described problems, the present disclosure is to provide smart wearable clothing that is excellent in a durability and conductivity and in which printing on a flexible fabric is possible.

Specifically, according to the present disclosure, an aspect is to provide smart wearable clothing with an excellent stretchability and a smart wearable system using the same by connecting a circuit electrode formed on a fabric to a chipset via a bond to a stretchable anisotropic conductive film (ACF).

However, goals to be achieved are not limited to those described above, and other goals not mentioned above can be clearly understood by one of ordinary skill in the art from the following description.

### TECHNICAL SOLUTIONS

Smart wearable clothing according to the present disclosure includes a fabric (sometimes, a substrate); a stretchable circuit electrode formed on the fabric; a stretchable ACF formed on the circuit electrode; and a chipset formed on the stretchable ACF.

According to an embodiment, the fabric may include at least one selected from a group consisting of cotton, nylon, polyester, polyethylene terephthalate (PET), polyethylene (PE), spandex, polyurethane (PU), neoprene, silk, artificial silk (art silk), rayon, modal, linen, wool, acrylic, artificial leather, laminated fabric, acetate, polypropylene, suede, neoprene, lyocell, high-density polyethylene (HDPE), and aramid.

According to an embodiment, the circuit electrode may be printed with a conductive ink.

According to an embodiment, the conductive ink may include at least one ink or paste selected from a group consisting of a particle-free metal salt solution, a carbon nanotube (CNT), graphene, a silver nano-particle, a silver nano-wire, a silver flake, a conductive polymer, and a metal particle.

According to an embodiment, a modulus of the conductive ink may be 10 times or less a modulus of the fabric.

According to an embodiment, a curing time of the conductive ink may range from 1 minute to 1 hour.

According to an embodiment, a strain rate of the circuit electrode may be 2 times or less based on a state in which an external force is not applied.

According to an embodiment, the stretchable ACF may include a thermoplastic rubber grafted with a maleic anhydride.

According to an embodiment, the thermoplastic rubber may include at least one selected from a group consisting of styrene-ethylene-butylene-styrene (SEBS), styrene-isoprene-styrene (SIS), styrene-butadiene-styrene (SBS), polyurethane (PU)-based rubber, and polyolefin (PO)-based rubber.

According to an embodiment, the stretchable ACF may be chemically or physically bonded to the fabric on which the circuit electrode is formed and the chipset.

According to an embodiment, the stretchable ACF may have a pitch of 1 micrometer (µm) to 200 µm.

According to an embodiment, the stretchable ACF may have a thickness of 1 µm to 200 µm.

According to an embodiment, the chipset may include at least one selected from a group consisting of an integrated circuit (IC), a light-emitting diode (LED), a system on chip (SoC), a transistor, a condenser, a capacitor, an antenna, and a sensor.

According to an embodiment, the smart wearable clothing may further include a heating wire printed on the fabric.

A smart wearable system according to the present disclosure includes the smart wearable clothing according to the present disclosure; a sensor formed on the stretchable ACF; and an ultra-lightweight device configured to supply a power to the sensor or perform wired or wireless data communication in real time.

According to an embodiment, the sensor may be a flexible sensor.

According to an embodiment, the wearable system may be customized for sports by sensing movements of joints and muscles or a heart rate and breathing by controlling a position and a combination of the sensor.

According to an embodiment, an application configured to control the ultra-lightweight device may be further included, and a condition or an exercise efficiency may be monitored in real time by connecting the sensor and an algorithm.

### EFFECTS OF THE INVENTION

To solve the above-mentioned problems, the present disclosure may provide smart wearable clothing that is excellent in a durability and conductivity and in which printing on a flexible fabric is possible.

Specifically, according to the present disclosure, smart wearable clothing with an excellent stretchability and a smart wearable system using the same by connecting a circuit electrode formed on a fabric to a chipset via a bond to a stretchable anisotropic conductive film (ACF) may be provided.

### BRIEF DESCRIPTION OF DRAWINGS

FIG. 1 is a diagram illustrating a concept of a configuration of smart wearable clothing according to an embodiment of the present disclosure.
FIG. 2 is a diagram illustrating a concept of a lamination structure of a cross section of smart wearable clothing according to an embodiment of the present disclosure.
FIG. 3 is a diagram illustrating a stretchable anisotropic conductive film (ACF) according to an embodiment of the present disclosure.
FIG. 4 is a diagram illustrating clothing with an applied smart wearable system according to an embodiment of the present disclosure.

### BEST MODE FOR CARRYING OUT THE INVENTION

Hereinafter, embodiments will be described in detail with reference to the accompanying drawings. However, various alterations and modifications may be made to the embodiments, and the embodiments are not meant to be limited by the descriptions of the present disclosure. The embodiments should be understood to include all changes, equivalents, and replacements within the idea and the technical scope of the disclosure.

The terminology used herein is for the purpose of describing particular embodiments only and is not intended to be limiting. The singular forms "a," "an," and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

In addition, when describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure. In addition, terms such as first, second, A, B, (a), (b), and the like may be used to describe components of the embodiments. Each of these terms is not used to define an essence, order or sequence of a corresponding component but used merely to distinguish the corresponding component from other component(s). It should be noted that if it is described in the specification that one component is "connected," "coupled" or "joined" to another component, the former may be directly "connected," "coupled," and "joined" to the latter or "connected", "coupled", and "joined" to the latter via another component.

A component, which has the same common function as a component included in any one embodiment, will be described by using the same name in other embodiments. Unless disclosed to the contrary, the description of any one embodiment may be applied to other embodiments, and the specific description of the repeated configuration will be omitted.

Smart wearable clothing according to the present disclosure includes a fabric; a stretchable circuit electrode formed on the fabric; a stretchable anisotropic conductive film (ACF) formed on the circuit electrode; and a chipset formed on the stretchable ACF.

The fabric may be a substrate, and in some examples, may be a flexible and stretchable substrate.

Since smart wearable clothing needs to be applied to soft fabric and deformed according to movements of joints or muscles of a body, a flexibility and elastic force are necessary. Conventional wearable clothing, which is a type of a wearable device attached to clothes, has a difficulty in application to flexible clothing, whereas, in the present disclosure, a device may be printed on a fabric and connected to an ACF, and thus, it is possible to provide stretchable smart wearable clothing that has a high adhesion to flexible clothing and also has a high durability and electrical conductivity.

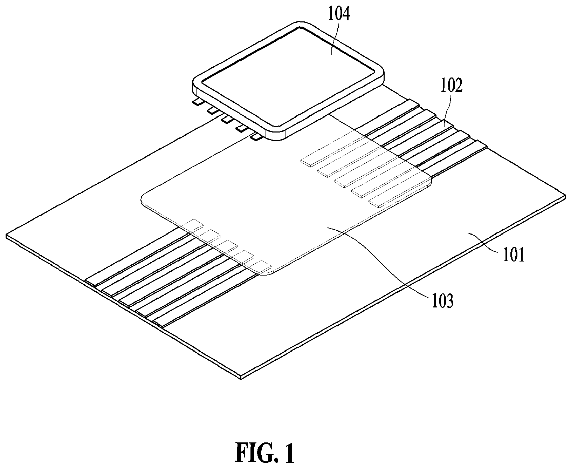

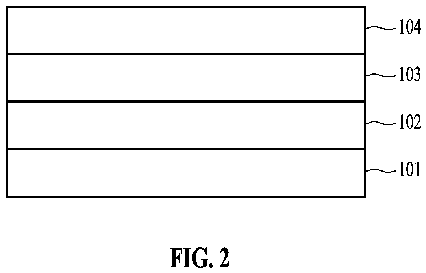

FIG. 1 is a diagram illustrating a concept of a configuration of smart wearable clothing according to an embodiment of the present disclosure. FIG. 2 is a diagram illustrating a concept of a lamination structure of a cross section of smart wearable clothing according to an embodiment of the present disclosure. However, FIG. 2 merely illustrates an example, and a thickness of each layer is not limited. Referring to FIGS. 1 and 2, the smart wearable clothing according to the present disclosure includes a fabric 101, a circuit electrode 102 formed on the fabric, a stretchable ACF 103 connected to the circuit electrode, and a chipset 104 connected to the stretchable ACF. The electrode 102 with a flexibility may be formed as a stretchable circuit electrode, and the electrode 102 and the chipset 104 may be electrically and physically connected via the stretchable ACF 103, to have a conductivity. When a flexible device is constructed by attaching the stretchable ACF 103, which is transparent and conducted in the vertical direction, in the same form as a sealing tape, and by completely attaching a desired electronic device to a desired area, high usefulness may be obtained. Thus, it is possible to provide a possibility of expanding from a conventional wearable device that is additionally attached to clothing to clothing itself that functions as a gear using a stretchable ACF.

According to an embodiment, the fabric may include at least one selected from a group consisting of cotton, nylon, polyester, polyethylene terephthalate (PET), polyethylene (PE), spandex, polyurethane (PU), neoprene, silk, artificial silk (art silk), rayon, modal, linen, wool, acrylic, artificial leather, laminated fabric, acetate, polypropylene, suede, neoprene, lyocell, high-density polyethylene (HDPE), and aramid.

The fabric may be for a variety of clothing such as military uniforms, leggings, buffs and sports clothing, or may be for industrial use such as high-density polyethylene (HDPE) and aramid among those listed above, and the fabric may include, but is not limited to, a fabric that requires a flexibility and a large number of movements.

According to an embodiment, the circuit electrode may be printed with a conductive ink.

To form a stretchable circuit electrode, printing on the fabric with the conductive ink may be performed. The conductive ink may be a flexible electronic material in the form of ink, and a circuit may be easily printed in a desired shape and size, because the ink itself conducts electricity. In addition, a flexible and elastic conductive ink may be folded and bent to fit a body and may form a stretchable circuit electrode that maintains a conductivity even when stretched, to be suitable for use in smart wearable clothing.

When a circuit electrode is formed with the conductive ink, the electrode may be printed on a fabric instead of separating and disconnecting the fabric itself and may have advantages of being economical and efficient as mass production is easy.

According to an embodiment, the conductive ink may include at least one ink or paste selected from a group consisting of a particle-free metal salt solution, a carbon nanotube (CNT), graphene, a silver nano-particle, a silver nano-wire, a silver flake, a conductive polymer, and a metal particle.

According to an embodiment, a modulus of the conductive ink may be 10 times or less than a modulus of the fabric. Desirably, the modulus of the conductive ink may be 8 times or less, and more desirably, 5 times or less the modulus of the fabric, however, the scope of the right of the present disclosure is not limited thereto. When the modulus of the conductive ink exceeds 10 times the modulus of the fabric, a problem of the conductive ink being separated from the fabric may occur. A problem does not occur despite a great difference of up to 10 times in modulus between the conductive ink and the fabric as described above, due to a chemical bond or strong physical bond between the fabric and the conductive ink.

According to an embodiment, a curing time of the conductive ink may range from 1 minute to 1 hour. Desirably, the curing time may range from 5 minutes to 30 minutes, however, the scope of the range of the present disclosure is not limited thereto. According to an embodiment, a curing process after printing with the conductive ink may be required to form a circuit electrode using the conductive ink. When the curing time of the conductive ink is less than 1 minute, a time for printing may be insufficient in reality, and when the curing time exceeds 1 hour, productivity may decrease.

According to an embodiment, a strain rate of the circuit electrode may be 2 times or less based on a state in which an external force is not applied. This indicates that restoration is possible even when it is doubled by the external force. When deformation is performed by 30% or greater (i.e., deformation by 130% or greater) based on the state in which the external force is not applied, electrical characteristics may be slightly reduced, but physical characteristics may remain unchanged and may be maintained up to 200%, and when the external force is removed, restoring to the original state may be performed, and the electrical characteristics may also be restored.

According to an embodiment, the circuit electrode may have high durability against physical shock and heat. Electrical and physical characteristics of the circuit electrode may be maintained even when repeatedly stretched 10,000 times or more, and the circuit electrode may not be damaged by heat below 230°C and the electrical and physical characteristics may be maintained.

According to an embodiment, the stretchable ACF may include a thermoplastic rubber grafted with a maleic anhydride.

The stretchable ACF may include a polymer film into which a conductive particle is inserted, may change in response to a deformation of a substrate due to an excellent flexibility thereof, may firmly bond interfaces of different members due to an excellent adhesion thereof, may include regularly arranged conductive particles to maintain a uniform and constant conductivity, and may freely control an area in which conductive particles are arranged.

According to an embodiment, the thermoplastic rubber grafted with the maleic anhydride may be excellent in a flexibility and resilience to be suitable as a material for the polymer film.

According to an embodiment, the thermoplastic rubber may include at least one selected from a group consisting of styrene-ethylene-butylene-styrene (SEBS), styrene-isoprene-styrene (SIS), styrene-butadiene-styrene (SBS), polyurethane (PU)-based rubber, and polyolefin (PO)-based rubber.

According to an embodiment, the stretchable ACF may be chemically or physically bonded to the fabric on which the circuit electrode is formed, and the chipset.

The stretchable ACF may change in response to a deformation of the substrate due to the excellent flexibility thereof, and may be firmly bonded to the chipset due to the excellent adhesion thereof while maintaining a deformed shape of the fabric on which the circuit electrode is formed, to maintain the conductivity. In other words, even when the fabric, which is a soft substrate, is stretched, the stretchable ACF, which is an intermediate layer, may assist in attaching a hard part onto the fabric while holding the fabric. Unlike conventional circuit electrodes and chipset electrodes that respectively correspond to each other, the stretchable ACF may be bonded, to maintain the conductivity and obtain an excellent stretchability.

According to an embodiment, the stretchable ACF may be chemically or physically bonded, and may be strongly bonded even at a low temperature and on a soft surface. Conventionally, it was not easy to attach devices, such as central processing units (CPUs), transistors, batteries, and the like, to flexible substrates and circuits, and accordingly, a melting and attaching method was used as a method of attachment during conduction. However, in this case, due to a difference in physical properties, problems of hardening a substrate together and a high possibility of delamination may occur. In the smart wearable clothing according to the present disclosure, chemically or physically bonding of a surface of the stretchable ACF treated with the maleic anhydride and a surface of the fabric or the chipset using the stretchable ACF, instead of a physical bond by melting and attachment, may be possible even at a low temperature, such as a temperature of 80°C to 100°C, and thus, damages of the chipset and the fabric may be minimized, and the chipset and the fabric may be attached.

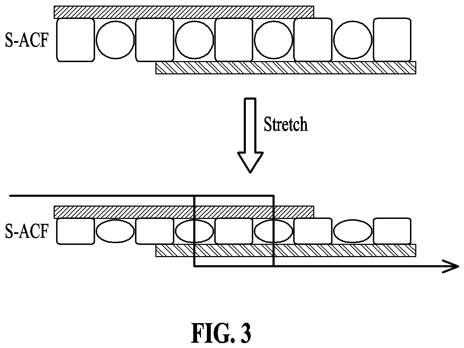

FIG. 3 is a diagram illustrating a stretchable ACF according to an embodiment of the present disclosure. Referring to FIG. 3, a stretchable ACF including a conductive particle may be connected to a fabric including a stretchable circuit electrode, and a chipset, and may have a flexibility as shown in FIG. 3 even when stretched, which may enable conduction between the circuit electrode and the chipset, and accordingly, the conductivity of the chipset, the circuit and the fabric connected to the stretchable ACF may be maintained so that wearable clothing may be folded and bent to fit a body.

According to an embodiment, the stretchable ACF may have a pitch of 1 micrometer (µm) to 200 µm. Desirably, the pitch may range from 5 µm to 100 µm, and from 10 µm to 50 µm, however, the right of the present disclosure is not limited thereto. The stretchable ACF may adjust the pitch to enable a precise circuit connection.

According to an embodiment, the stretchable ACF may be in the form of a semi-transparent layer, and if the stretchable ACF is widely attached to the desired area unlike connecting of an adhesive portion to an intermediate portion to correspond to each electrode in the related art, conduction in the vertical direction may be possible due to a pitch of the stretchable ACF less than a size of an electrode needing to correspond.

According to an embodiment, the stretchable ACF may have a thickness of 1 µm to 200 µm. Desirably, the thickness may range from 5 µm to 100 µm, and from 10 µm to 50 µm, however, the right of the present disclosure is not limited thereto. When the thickness is less than or equal to 1 µm, a problem of electrical and physical reliability may occur, and when the thickness exceeds 200 µm, a problem with an electrical resistance or a conductivity through a conductive ball may occur.

The stretchable ACF may adjust the pitch to enable a precise circuit connection.

According to an embodiment, the chipset may include at least one selected from a group consisting of an integrated circuit (IC), a light-emitting diode (LED), a system on chip (SoC), a transistor, a condenser, a capacitor, an antenna, and a sensor. In an example, a connection of circuit electrode-ACF-chipset may be possible, and a connection of circuit electrode-ACF-circuit electrode may also be possible. A circuit connection may be broken during a weaving process (sewing, stitching, etc.) between fabrics, and in this case, the ACF may exhibit a function of electrically connecting (conducting) the fabric.

The chipset may be electrically connected to the circuit electrode to perform a function of an electronic device to be used as smart wearable clothing, and may function to emit light or sense information.

According to an embodiment, the smart wearable clothing may further include a heating wire printed on the fabric.

According to an embodiment, the heating wire may be printed with conductive ink. The heating wire may have a heating function of generating heat by increasing voltage, and the temperature may be controlled by adjusting the voltage. The heating wire may be printed on the fabric, may be lightweight and may have high mobility in comparison to an existing system, and may be disposed on the same surface of the fabric unlike general heating wires so that there is no feeling of physical contact with skin.

When the smart wearable clothing has a flexibility, wearable clothing that maintains the conductivity and shape despite deformation due to stretching and a movement of a body may be provided.

A smart wearable system according to the present disclosure may include the smart wearable clothing according to the present disclosure; a sensor formed on the stretchable ACF; and an ultra-lightweight device configured to supply a power to the sensor or perform wired or wireless data communication in real time.

According to an embodiment, the sensor may be a flexible sensor.

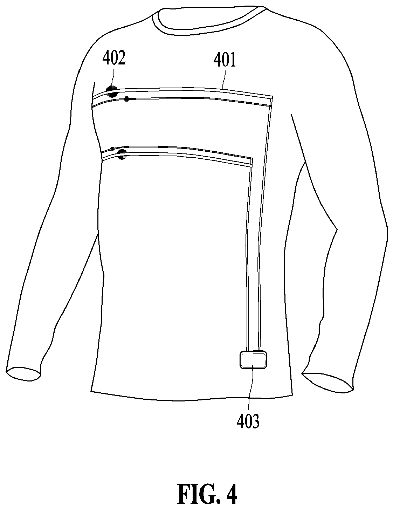

FIG. 4 is a diagram illustrating clothing with an applied smart wearable system according to an embodiment of the present disclosure. Referring to FIG. 4, an example of clothing constructed by connecting smart wearable 402, which includes a fabric, a circuit electrode, a stretchable ACF, and a (flexible) sensor according to the present disclosure, to an ultra-lightweight device 403 by a circuit electrode 401 may be confirmed.

According to an embodiment, the sensor may be a pressure and strain sensor that is printable and that has a simple structure. It may be a metal-polymer compound, may have a high flexibility, and may be manufactured in various sizes and shapes through screen/nozzle printing.

According to an embodiment, the ultra-lightweight device may be rechargeable, may be magnetically attachable and detachable, and may be an ultra-small wired or wireless data transmission system, that is, a wired or wireless power transmission system that may be applied to various sports with a single device, and may be a power transmission and wireless charging auxiliary battery based on a POGO connector, a cable-type wiring, and the like.

Unlike general wearables, the above smart wearable system in the form of clothing may be applicable to sports clothing, functional clothing, general clothing, and the like, without restrictions on the shape, and may be applicable in fields, such as sports, healthcare, metaverse, and the like.

According to an embodiment, the wearable system may be customized for sports by sensing movements of joints and muscles or a heart rate and breathing by controlling a position and a combination of the sensor.

The wearable system, which is a sports/fitness smart wearable system including a flexible circuit and a sensor, may monitor an exercise status and metabolism in real time and may enter fields of sports in which a measurement and analysis with cameras are impossible. The sensor may be a strain sensor, and may be connected to a circuit including a stretchable electrode to analyze a movement of a muscle and a joint range of motion in real time, which may facilitate identification of the exercise status and management of injuries.

According to an embodiment, an application configured to control the ultra-lightweight device may be further included, and a condition or an exercise efficiency may be monitored in real time by connecting the sensor and an algorithm.

According to an embodiment, the smart wearable system may be properly applied to each sport to measure metabolism using a combination of various flexible sensors and may monitor an exercise efficiency and condition of a player in real time in conjunction with an algorithm.

For example, when playing golf, a driving distance may be identified while an exercise status is monitored, a heating wire of a wearable may be activated and the level of heating may be adjusted according to the season. When playing soccer, a heart rate, a breathing rate, and an amount of exercise of a player may be measured in real time to monitor an activity, an exercise efficiency, and metabolism. In addition, in a fitness situation, the exercise effect may be efficiently monitored by checking a muscle mass, an exercise time, and body fit before and after exercise.

According to an embodiment, the smart wearable system may allow current to flow through the smart wearable clothing according to the present disclosure formed in a desired area, to enable an in-body measurement and identify the in-body measurement in the application.

Hereinafter, the present disclosure will be described in detail with reference to the following example and comparative example.

However, the following example is only for illustrating the present disclosure, and the description of the present disclosure is not limited to the following example.

### Example

A fabric including a circuit electrode printed with a conductive ink was manufactured, and a stretchable ACF including SEBS grafted with a maleic anhydride was prepared, to perform a test analysis according to a method described below.

### Analysis of Physical and Electrical Characteristics of Circuit Electrode

After measuring an initial resistance of the circuit electrode printed with the conductive ink on a fabric substrate, a real-time resistance variation was measured by applying deformation of stretching, bending, twisting, and folding to a substrate including an electrode. Here, an elongation rate up to a point where the electrode loses characteristics thereof was set as the maximum elongation rate of the electrode, and a durability of the electrode was evaluated according to the number of cycles up to the point. A difference in surface shape before and after the deformation was compared and analyzed using an optical microscope and a scanning electron microscope (SEM), and a stress/strain curve was obtained according to a test analysis method through UTM, to confirm the physical characteristics of the electrode and the substrate.

### Analysis of Physical and Electrical Characteristics of Circuit Electrode and ACF

After bonding the circuit electrode and the ACF, the initial resistance between the circuit electrode-ACF-circuit electrode was measured. The circuit electrode-ACF-circuit electrode were deformed by stretching, bending, twisting, and folding, and the real-time resistance variation was measured, and whether conduction occurs between respective electrode lines connected to the ACF was determined. The adhesive strength of circuit electrode-ACF-circuit electrode was measured through UTM.

### Evaluation of Reliability of Circuit Electrode and ACF

After a high-temperature and high-humidity evaluation and a thermal shock evaluation (repeated cycle of a low temperature and high temperature) were performed on the ACF and the circuit electrode printed on the fabric substrate, whether electrical conduction occurs was determined.

As a result of the above test, it may be confirmed that excellent electrical characteristics of the circuit electrode and the ACF are maintained even under various physical deformations.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order and/or if components in a described system, architecture, device, or circuit are combined in a different manner and/or replaced or supplemented by other components or their equivalents.

Therefore, other implementations, other embodiments, and equivalents to the claims are also within the scope of the following claims.

## Claims

1. Smart wearable clothing comprising:
a fabric;
a stretchable circuit electrode formed on the fabric;
a stretchable anisotropic conductive film (ACF) formed on the circuit electrode; and
a chipset formed on the stretchable ACF.

2. The smart wearable clothing of claim 1, wherein the fabric comprises at least one selected from a group consisting of cotton, nylon, polyester, polyethylene terephthalate (PET), polyethylene (PE), spandex, polyurethane (PU), neoprene, silk, artificial silk (art silk), rayon, modal, linen, wool, acrylic, artificial leather, laminated fabric, acetate, polypropylene, suede, neoprene, lyocell, high-density polyethylene (HDPE), and aramid.

3. The smart wearable clothing of claim 1, wherein the circuit electrode is printed with a conductive ink.

4. The smart wearable clothing of claim 3, wherein the conductive ink comprises at least one ink or paste selected from a group consisting of a particle-free metal salt solution, a carbon nanotube (CNT), graphene, a silver nano-particle, a silver nano-wire, a silver flake, a conductive polymer, and a metal particle.

5. The smart wearable clothing of claim 3, wherein a modulus of the conductive ink is 10 times or less than a modulus of the fabric.

6. The smart wearable clothing of claim 3, wherein a curing time of the conductive ink ranges from 1 minute to 1 hour.

7. The smart wearable clothing of claim 1, wherein a strain rate of the circuit electrode is 2 times or less based on a state in which an external force is not applied.

8. The smart wearable clothing of claim 1, wherein the stretchable ACF comprises a thermoplastic rubber grafted with a maleic anhydride.

9. The smart wearable clothing of claim 8, wherein the thermoplastic rubber comprises at least one selected from a group consisting of styrene-ethylene-butylene-styrene (SEBS), styrene-isoprene-styrene (SIS), styrene-butadiene-styrene (SBS), polyurethane (PU)-based rubber, and polyolefin (PO)-based rubber.

10. The smart wearable clothing of claim 1, wherein the stretchable ACF is chemically or physically bonded to the fabric on which the circuit electrode is formed, and the chipset.

11. The smart wearable clothing of claim 1, wherein the stretchable ACF has a pitch of 1 micrometer (µm) to 200 µm.

12. The smart wearable clothing of claim 1, wherein the stretchable ACF has a thickness of 1 µm to 200 µm.

13. The smart wearable clothing of claim 1, wherein the chipset comprises at least one selected from a group consisting of an integrated circuit (IC), a light-emitting diode (LED), a system on chip (SoC), a transistor, a condenser, a capacitor, an antenna, and a sensor.

14. The smart wearable clothing of claim 1, wherein the smart wearable clothing further comprises a heating wire printed on the fabric.

15. A smart wearable system comprising:
the smart wearable clothing of claim 1;
a sensor formed on the stretchable ACF; and
an ultra-lightweight device configured to supply a power to the sensor or perform wired or wireless data communication in real time.

16. The smart wearable system of claim 15, wherein the sensor is a flexible sensor.

17. The smart wearable system of claim 15, wherein the wearable system is customized for sports by sensing movements of joints and muscles or a heart rate and breathing by controlling a position and a combination of the sensor.

18. The smart wearable system of claim 15, further comprising:
an application configured to control the ultra-lightweight device,
wherein a condition or an exercise efficiency is monitored in real time by connecting the sensor and an algorithm.
